(19) Europäisches Patentamt
European Patent Office
Office européen des brevets

(11) **EP 3 191 813 B1**

(12) **FASCICULE DE BREVET EUROPEEN**

(45) Date de publication et mention
de la délivrance du brevet:
**27.05.2020  Bulletin 2020/22**

(51) Int Cl.:
***G01N 3/08*** *(2006.01)*      ***G01N 3/32*** *(2006.01)*
***G01N 33/44*** *(2006.01)*

(21) Numéro de dépôt: **15766920.1**

(22) Date de dépôt: **04.09.2015**

(86) Numéro de dépôt international:
**PCT/FR2015/052355**

(87) Numéro de publication internationale:
**WO 2016/038284 (17.03.2016 Gazette 2016/11)**

(54) **MACHINE D'ESSAI POUR CONTRÔLE QUALITÉ D'UNE PIÈCE AU MOINS PARTIELLEMENT EN ÉLASTOMÈRE CHARGÉ**

PRÜFMASCHINE ZUR QUALITÄTSKONTROLLE EINES ZUMINDEST TEILWEISE AUS GEFÜLLTEM ELASTOMER HERGESTELLTEN BAUTEILS

TESTING MACHINE FOR QUALITY CONTROL OF A COMPONENT AT LEAST PARTIALLY MADE OF FILLED ELASTOMER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.09.2014  FR 1458411**

(43) Date de publication de la demande:
**19.07.2017  Bulletin 2017/29**

(73) Titulaire: **SumiRiko SD France S.A.S.
58300 Decize (FR)**

(72) Inventeurs:
  • VERGER, Serge
    **58260 La Machine (FR)**
  • LABAUNE, Philippe
    **03400 Yzeure (FR)**
  • PANNIER, Stéphane
    **03000 Moulins (FR)**
  • CHARNOTET, Thierry
    **58300 Saint Leger des Vignes (FR)**

(74) Mandataire: **Ernest Gutmann - Yves Plasseraud S.A.S.
66, rue de la Chaussée d'Antin
75009 Paris (FR)**

(56) Documents cités:
**WO-A1-2012/080675      FR-A1- 2 925 691**

• **COVENEY V A ET AL: "A Triboelastic Model for the Cyclic Mechanical Behavior of Filled Vulcanizates", RUBBER CHEMISTRY AND TECHNOLOGY, AMERICAN CHEMICAL SOCIETY, RUBBER DIVISION, US, vol. 68, no. 4, 1 septembre 1995 (1995-09-01), pages 660-670, XP008176143, ISSN: 0035-9475, DOI: 10.5254/1.3538765**
• **COVENEY V A ET AL: "Rate-dependent modeling of a highly filled vulcanizate", RUBBER CHEMISTRY AND TECHNOLOGY, AMERICAN CHEMICAL SOCIETY, RUBBER DIVISION, US, vol. 73, no. 4, 1 septembre 2000 (2000-09-01), pages 565-577, XP008176142, ISSN: 0035-9475, DOI: 10.5254/1.3547606**

**EP 3 191 813 B1**

**Description**

Domaine technique

La présente invention concerne le domaine technique des procédés de contrôle qualité d'une pièce fabriquée. Plus précisément, la présente invention concerne le domaine technique des procédés de contrôle qualité d'une pièce fabriquée au moins en partie en élastomère chargé, en particulier en caoutchouc.

État de la technique et problème à la base de l'invention

Dans le domaine automobile par exemple, il est important de procéder à des contrôles qualité des pièces fabriquées, notamment pour garantir la qualité/conformité des pièces. De manière générale, la fabrication de pièces au moins en partie en élastomère chargé doit répondre à un cahier des charges particulier. Ce cahier des charges définit des caractéristiques que doit présenter la pièce, telle que la mesure de la raideur et de la phase pour déterminer l'amortissement (ou *loss angle* en anglais) pour une ou plusieurs sollicitations mécaniques.

Les caractéristiques que l'on cherche à déterminer peuvent être par exemple des caractéristiques quasi-statiques. Pour cela, des méthodes de détermination de ces caractéristiques mettent en oeuvre des essais en régime quasi-statique, c'est-à-dire qu'un effort est progressivement appliquée sur la pièce pour la déformer. La vitesse de déformation de la pièce en régime quasi-statique est fréquemment de l'ordre de 10 mm/min. Ces méthodes utilisent généralement une machine de traction-compression. Dans ce type de machine, afin d'étudier le comportement de la pièce face à un effort axial, ou radial, conique, de torsion, la pièce à analyser est maintenue fixée à un banc de test de la machine de traction-compression et à un vérin de celle-ci. Le vérin est déplaçable en translation de manière à exercer successivement sur la pièce un effort en compression et un effort en traction. La déformation de la pièce (généralement mesurée par la hauteur de la pièce parallèlement à la direction de l'effort appliqué) et la charge appliquée sont mesurées pendant les phases de compression et de traction afin d'obtenir une courbe de transfert effort/déformation. La raideur et l'amortissement sont alors déterminés à partir de ces mesures.

Du fait de la nécessité de fixer la pièce à des éléments de la machine de traction-compression pour pouvoir appliquer la succession d'efforts en compression et en traction, ces méthodes sont relativement longues, généralement supérieur à 10 min pour chaque pièce soumise à la mesure. Elles nécessitent par ailleurs un personnel qualifié pour correctement disposer la pièce dans la machine et conduire le test. Ainsi, ces méthodes ne peuvent être implémentées dans une ligne de fabrication afin de pouvoir contrôler la qualité de toutes les pièces qui sont fabriquées pour des raisons de cadence ; un prélèvement d'échantillons étant nécessaire pour pouvoir les fixer sur la machine de traction-compression.

En outre, le cahier des charges nécessite aussi parfois la vérification de la conformité des caractéristiques mécaniques en dynamique pour une ou plusieurs amplitudes de déformation et une ou plusieurs fréquences. Ces mesures s'effectuent conventionnellement également à l'aide de machines de traction-compression servo-hydrauliques. Une contrainte est appliquée à la pièce de manière à ce que la courbe de déformation d'une région témoin de la pièce décrive une sinusoïde. La fréquence de la sinusoïde est généralement supérieure ou égale à 1 Hz. Ainsi, il est d'autant plus nécessaire de fixer la pièce pour qu'elle puisse suivre cette fréquence de déformation.

Aussi, les machines de traction-compression servo-hydrauliques sont relativement onéreuses.

À titre d'information soit à l'encontre de la personne ayant commandé les pièces, soit à l'encontre de l'utilisateur final, il est parfois nécessaire d'obtenir ces caractéristiques dynamiques pour plusieurs fréquences et amplitudes maximales de déformation. Ainsi, les étapes mentionnées ci-dessus sont réitérées pour plusieurs amplitudes de déformation et plusieurs fréquences d'application des efforts différents.

Par ailleurs, toutes ces méthodes sont plus coûteuses en termes d'énergie, qu'elle soit due à la consommation d'huile, d'électricité ou d'eau par exemple. Elles nécessitent également des opérateurs qualifiés et sont bruyantes. Ainsi, le coût général pour effectuer ces mesures est relativement élevé.

Le document WO 2012/080675 a pour objet un procédé de pilotage de façon quasi-statique et modulée d'un dispositif d'essais de sollicitations mécaniques sur un échantillon réalisé essentiellement à partir d'un ou plusieurs matériaux présentant des comportements viscoélastoplastiques, permettant la caractérisation du comportement mécanique de l'échantillon et de la modélisation de ce comportement. Ce dispositif comprend au moins un ensemble de sollicitation mécanique comprenant au moins un actionneur permettant d'entraîner un moyen de préhension en déplacement axial, suivant un axe de l'échantillon maintenu en position de sollicitation. Il consiste essentiellement, à partir d'un ordinateur, à commander le ou chaque actionneur en envoyant et en injectant dans ce dernier un signal représentatif d'une consigne de vitesse ou de force, ledit signal étant un signal modulé sinusoïdal v(t) comportant une composante quasi-statique et une composante dynamique sinusoïdale. Le document WO 2012/080675 a également pour objet un dispositif d'essais convenant à la mise en oeuvre de ce procédé.

L'état de la technique comprend également le document FR 2 925 691 et les articles de COVENEY V A et Al :

« A triboelastic Model for the Syclic Mechanical Behavior of Filled Vulcanizates » et « Rate-dependent modeling of a highly filled vulcanizate » publiés dans RUBBER CHEMISTRY AND TECHNOLOGY, AMERICAN SOCIETY, RUBBER DIVISION, US, respectivement volume 68, n°4, 1er septembre 1995, pages 660-670 et volume 73, n°4, 1er septembre 2000, pages 565-577.

Il existe par conséquent le besoin d'un procédé de caractérisation du comportement mécanique de pièces soumises à des sollicitations qui puisse être mise en œuvre directement dans une ligne de fabrication de telles pièces.

Présentation de l'invention

Un exposé de l'invention est donné ci-après.

L'un des objectifs de l'invention est de pallier à au moins un des inconvénients de l'état de la technique présentés ci-dessus.

L'invention propose une machine d'essai selon la revendication 1.

Par rapport à l'état de la technique antérieure, les avantages de l'invention résident dans le fait que seule l'application d'un effort en compression sur la pièce est nécessaire afin de permettre le contrôle qualité de celle-ci. Par ailleurs, cela permet en outre de supprimer la nécessité de fixation de la pièce dans la machine d'essai. Par conséquent, le temps nécessaire à la détermination de la conformité de la pièce au cahier des charges est réduit, ce qui permet l'implémentation du contrôle qualité unitaire dans une ligne de fabrication de la pièce.

Présentation des dessins

Les figures des dessins sont décrites ci-après. Ces figures sont données à titre illustratif et non-limitatif, parmi lesquels :

- la figure 1 est un organigramme illustrant les étapes du procédé ne formant pas partie de l'invention ;

- la figure 2 est une représentation schématique de l'étape d'accommodation du procédé de la figure 1 où l'on peut voir une pièce constituée d'un élastomère chargé **P** et de deux armatures métalliques **1, 2,** disposée sans fixation dans un logement de la machine d'essai spécialement prévu à cet effet, et un vérin **3** pour l'application d'une force de compression ;

- la figure 3 est une représentation schématique de l'étape d'application d'une force de compression progressive et de l'étape de mesure de la force de compression appliquée et de la déformation pour le procédé de la figure 1, où l'on peut voir une pièce constituée d'un élastomère chargé **P** et de deux armatures métalliques **1, 2,** disposée sans fixation dans un logement de la machine d'essai spécialement prévu à cet effet, et un vérin **3** pour l'application d'une force de compression ;

- la figure 4 est un exemple de représentation graphique des mesures effectuées, elle montre un graphique représentant la force de compression appliquée en fonction du déplacement ;

- la figure 5 est un exemple du résultat pouvant être obtenu avec le procédé de la figure 1, elle représente l'effort estimé subi en fonction du déplacement représentée de la manière classiquement utilisée pour représenter le résultat obtenu avec une méthode conventionnelle dans laquelle la pièce est soumise à plusieurs cycles comprenant l'application d'une force de compression et l'application d'une force de traction sur la pièce pour plusieurs amplitudes de traction/compression ;

- la figure 6 est une représentation schématique d'une cellule élémentaire utilisée dans un modèle STS pour la pièce en partie en élastomère chargé ;

- la figure 7 est une représentation schématique d'une cellule élémentaire utilisée dans un modèle RT (triboélastique dépendant de la vitesse) pour la pièce en partie en élastomère chargé ;

- la figure 8 est une représentation schématique de la pièce en partie en élastomère chargé utilisée dans un modèle STS avec N cellules élémentaires ;

- la figure 9 est une représentation schématique du modèle STS équivalent de la figure 8 avec $C_T = 2K_T F_S$ ;

- la figure 10 représente schématiquement une machine d'essai spécialement dédiée au procédé ne formant pas partie de l'invention ; et

- la figure 11 représente schématiquement une ligne de fabrication et de pièces au moins partiellement en élastomère chargé selon l'invention comprenant la machine d'essai de la figure 10 dans une station de contrôle qualité.

Description de l'invention

Un exposé détaillé de plusieurs modes de réalisation de l'invention assorti d'exemples et de référence aux dessins est donné ci-après.

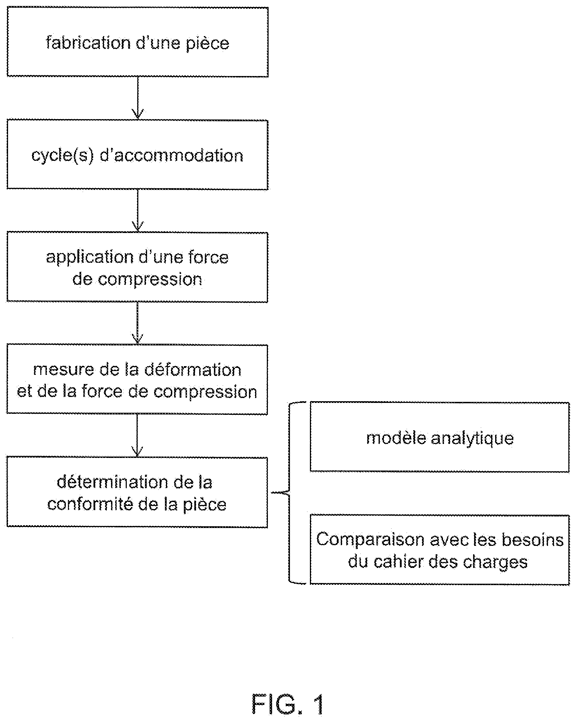

Tout d'abord, un procédé de contrôle qualité d'une pièce au moins partiellement en élastomère chargé ne formant pas partie de l'invention est décrit ci-après en référence aux figures 1 à 8. Ce procédé permet particulièrement le contrôle qualité de pièces au moins partiellement en élastomère chargé, par exemple du caoutchouc, tel que typiquement des pièces pour application automobile, ferroviaire, etc.

Par le terme « élastomère chargé » on entend dans le cadre de la présente invention un polymère chargé par des charges renforçantes, par exemple du noir de carbone ou un mélange de silice et de silane, présentant des propriétés élastiques obtenues après réticulation, par exemple le caoutchouc, le polybutadiène, le copolymère styrène-butadiène, le polyisobutylène (ou encore isobutylène-isoprène, aussi appelé caoutchouc butyle), etc.

Par le terme « caoutchouc » on entend à la fois les caoutchoucs naturels et les caoutchoucs synthétiques. Les caoutchoucs naturels sont fabriqués à partir de latex extrait de plantes telles que par exemple l'hévéa (*Hevea brasiliensis*) et le caoutchouc (*Ficus elastica*). Les caoutchoucs synthétiques sont fabriqués à partir de monomères issus de combustibles fossiles.

On entend également par le terme « caoutchouc » les mélanges faits au moins d'un caoutchouc naturel et au moins d'un caoutchouc synthétique, les caoutchoucs naturels chargés, les caoutchoucs synthétiques chargés, les mélanges chargés, ainsi que les composites composés principalement de caoutchouc naturel et/ou synthétique chargé.

Par composite, on comprendra des matériaux composés d'au moins deux matières comprenant des propriétés physiques et/ou chimiques significativement différentes qui sont combinées de manière à ce que le composite obtenu présente des caractéristiques différentes des matières qui le composent (appelées composants). Les composants ne sont pas mélangés et demeurent distincts les uns des autres tout en étant en contact intime entre eux, c'est-à-dire qu'ils sont difficilement séparables les uns des autres.

Le procédé comprend :

a) la réalisation d'au moins un cycle d'accommodation de la pièce, le cycle d'accommodation comprenant l'application d'une force de compression progressive sur la pièce et le relâchement progressif sans traction de la force de compression (figure 1) ;

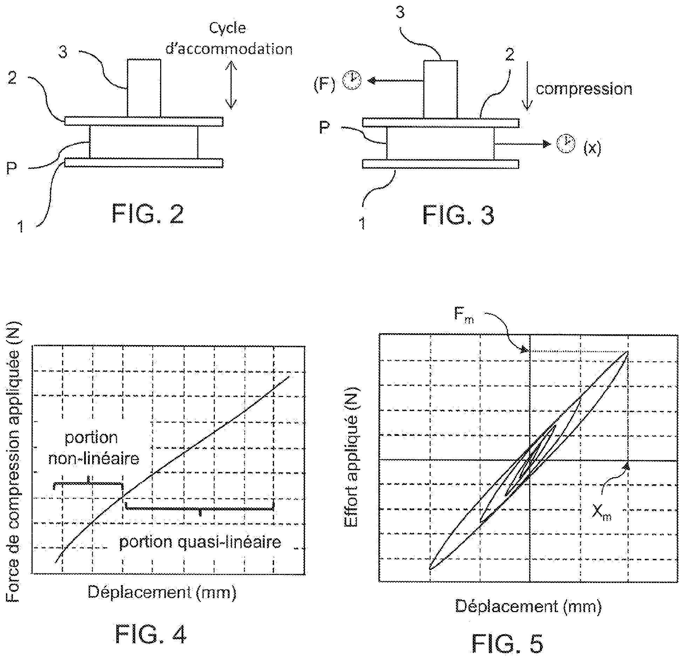

b) l'application d'une force de compression progressive sur la pièce en régime quasi-statique selon un profil prédéterminé de déformation de la pièce donnée (figure 2) ;

c) la mesure de la déformation de la pièce et de la force de compression appliquée pendant l'application de la force de compression (figure 3) ;

d) la détermination de la conformité de la pièce sous une autre sollicitation que celle de la force de compression de l'étape b) à partir de la mesure de la déformation de la pièce et de la force de compression appliquée, la conformité étant déterminée selon le modèle du solide triboélastique standard.

Ainsi, cette méthode ne fait appelle à aucun effort en traction. Ceci permet de s'affranchir de la nécessité de fixer la pièce dans la machine d'essai.

Le cycle d'accommodation permet de s'affranchir de l'effet Mullins. L'effet Mullins est un aspect particulier de la réponse mécanique des élastomères, dans lequel la courbe effort/déformation dépend de la charge maximale qui a été précédemment appliquée. Il se manifeste par une perte de rigidité pour des déformations appliquées après une première sollicitation.

Bien qu'un seul cycle d'accommodation soit nécessaire, il est parfois préférable de réaliser deux ou trois cycles d'accommodation, voire plus, afin de mieux stabiliser l'élastomère avant de réaliser les mesures.

Par les termes « déformation de la pièce », on entend dans le présent exposé la déformation qui se produit dans une région témoin de la pièce. Cette région témoin est l'endroit sur la pièce où la force est appliquée. L'homme du métier saura déterminer cette région témoin et ainsi mesurer la déformation de la pièce.

Le terme « profil de déformation » doit être compris dans l'ensemble du présent exposé comme désignant une évolution temporaire de la déformation de la pièce. La dérivée du profil de déformation donne la vitesse de déformation.

Le profil de déformation est avantageusement quasi-linéaire. Par ce terme « quasi-linéaire », on comprend dans l'ensemble de la présente invention une vitesse de déformation qui varie de ±5% d'une valeur choisie, voire qui est constante à la valeur choisie (profil linéaire). De préférence, la vitesse de déformation, en valeur absolue, de la pièce lors de la compression et du relâchement réalisés à l'étape a) doit être supérieure à la vitesse de déformation de la pièce lors de la compression réalisée lors de l'étape b) pour optimiser le temps de cycle d'accommodation.

Alternativement, la vitesse de déformation, en valeur absolue, de la pièce lors de la compression et du relâchement réalisés à l'étape a) et celle de la compression réalisée lors de l'étape b) peuvent être égales

Par ailleurs, l'amplitude de la déformation, par rapport au 0, lors de l'étape a) doit être de préférence supérieure à celle utilisée lors de l'étape b). En pratique, elle peut être égale à la valeur maximale pour laquelle la détermination de la conformité de la pièce doit être effectuée.

Dans un autre exemple, le profil de déformation peut aussi être linéaire par partie, c'est-à-dire qu'il présente des portions sur lesquelles il est linéaire et qu'à l'intersection de ces portions, la vitesse de déformation change. Le profil de déformation peut encore être courbe, périodique (triangle ou sinusoïdal, etc.), etc.

Par l'adjectif « progressif », on entend que la force de compression appliquée est au départ faible et qu'elle est ensuite augmentée petit à petit, de préférence continûment, bien que dans un temps très court, ou que le relâchement de la force appliquée est réalisé petit à petit, de préférence continûment.

Le régime quasi-statique désigne un régime dans lequel, bien que la force de compression appliquée change et que la pièce se déforme tout au long de l'application de la force de compression, le comportement de la pièce à chaque instant considéré individuellement de la compression se rapproche d'un comportement statique. C'est-à-dire que les phénomènes physiques qui interviennent uniquement dans un régime dynamique sont minimisés.

De préférence, le régime quasi-statique est caractérisé par un temps d'application de la force de compression de moins d'une second, de préférence à une vitesse de déformation de la pièce, définie dans l'ensemble du présent exposé dans le sens de la force de compression appliquée inférieure à 1 mm/s, de préférence supérieure à 10 mm/min.

La force de compression est avantageusement appliquée jusqu'à atteindre une amplitude maximale de déformation de la pièce se situant dans un domaine où la relation entre la force appliquée et la déformation de la pièce est linéaire et hors de la limite entre le domaine non-linéaire et le domaine linéaire. En effet, comme il a été mentionné ci-dessus, lorsque l'amplitude maximale d'une force appliquée est inférieure à l'amplitude maximale d'une force précédemment appliquée l'élastomère chargé a un comportement tout d'abord non-linéaire jusqu'à ce que la force appliquée atteigne une amplitude donnée à partir de laquelle le comportement de l'élastomère chargé devient linéaire.

De manière générale, après le ou les cycles d'accommodation, il suffit d'appliquer la force de compression sur la pièce sans relâchement et d'effectuer une seule série de mesures lors de l'application de la force de compression. Néanmoins, il est possible également d'effectuer un relâchement, sans traction, à la suite de l'application de la force de compression formant un cycle de mesure, la mesure étant alors réalisée pendant tout le cycle. Plusieurs cycles de mesure peuvent être aussi effectués, la mesure de la force de compression appliquée et de la déformation de la pièce étant réalisée pendant ces plusieurs cycles.

Un graphe peut être tracé à partir des mesures de la force appliquée et de la déformation de la pièce afin d'obtenir une représentation visuelle du comportement de la pièce par rapport à la force appliquée, bien que son tracé ne soit pas obligatoire pour la suite du procédé. Communément, la déformation est représentée en abscisses et la force appliquée en ordonnées. Il sera alors observé une première portion de courbe non-linéaire jusqu'à ce que la déformation (et donc également la force de compression appliquée) atteigne une valeur limite. Si on continue à augmenter la force de compression, une deuxième portion de courbe linéaire pourra alors être observée. Une telle portion de courbe possède donc une équation de la forme :

$$F(x) = p.x + a \; ;$$

avec $F(x)$ la force de compression appliquée, $x$ la déformation, $p$ la pente de la courbe, pouvant varier en fonction de $x$ de ± 5 %, et $a$ l'abscisse à l'ordonnée.

Les termes « autre sollicitation » désignent un effort mécanique différent de celui appliqué à l'étape b). Ainsi, le présent procédé permet de déterminer la conformité de la pièce à une sollicitation qui ne lui a pas été appliquée.

L'autre sollicitation peut être l'application d'une force de compression à un profil de déformation de la pièce différent de celui utilisé pour l'étape b).

L'autre sollicitation peut encore être une sollicitation dynamique contrairement à la force de compression appliquée à l'étape b) qui est une sollicitation quasi-statique. La sollicitation dynamique est généralement l'application répétée d'une force de compression et d'une force de traction. Cette application répétée est généralement périodique, par exemple sinusoïdale ou triangle, et présente donc une fréquence donnée.

La détermination de la conformité de la pièce consiste à vérifier que les propriétés techniques de la pièce sont répondent aux besoins, par exemple ceux exprimés dans un cahier des charges. Elle comprend avantageusement la détermination de la valeur d'au moins une caractéristique mécanique quasi-statique intrinsèque, de préférence de deux caractéristiques mécaniques quasi-statiques intrinsèques, de la pièce relative à sa réponse à un chargement quasi-statique, et la comparaison de cette valeur, de préférence ces valeurs, à une valeur prédéterminée traduisant notamment les besoins d'un cahier des charges.

Dans le cas où les valeurs de deux caractéristiques mécaniques quasi-statiques intrinsèques sont déterminées, ces deux caractéristiques mécaniques sont avantageusement la raideur et la phase en régime stationnaire, appelées ci-après raideur quasi-statique et phase quasi-statique.

La détermination de la caractéristique mécanique, notamment de la raideur quasi-statique et de la phase quasi-statique, effectuée grâce au procédé ne formant pas partie de l'invention aboutit à des valeurs très proches de celles qui auraient été obtenues par des méthodes plus conventionnelles (voir exemple ci-dessous), comme par exemple des essais réalisés à l'aide d'une machine de traction-compression. En effet, les mêmes résultats de raideur quasi-statique et de phase quasi-statique que pour un procédé utilisant une machine de traction-compression, dans lequel un cycle d'application d'une force de compression et d'une force de traction est répété plusieurs fois (avec une vitesse de déformation généralement inférieure à 10 mm/min) et où la force appliquée et la déformation de la pièce sont mesurées, peuvent être obtenus.

La caractéristique mécanique, notamment la raideur quasi-statique et/ou la phase quasi-statique, est par exemple déterminée à partir d'un même modèle analytique effort/déformation pour des pièces du type de la pièce, et des mesures obtenues à l'étape c).

Le modèle analytique effort/déformation permet éventuellement d'obtenir une courbe de transfert représentant la force de compression estimée nécessaire à être appliquée en fonction de la déformation. Cette courbe de transfert correspond à celle qu'on aurait obtenue en quasi-statique avec les méthodes conventionnelles qui est obtenue à partir des mesures effectuées. Cette courbe de transfert décrit généralement un cycle d'hystérésis, c'est-à-dire un contour fermé, généralement symétrique par rapport à l'origine, i.e. le point (0 ; 0). La courbe de transfert n'est pas nécessairement dessinée ou affichée.

Un exemple de modèle analytique de déformation est le modèle du solide triboélastique standard (en anglais *standart triboelastic solid model* ou encore *STS model*). Ce modèle est un modèle analytique décrit dans le document intitulé « A triboelastic model for the cyclic mechanical behavior of filled vulcanizates » (en français « Un modèle triboélastique pour le comportement mécanique cyclique des vulcanisés chargés ») de V. A. Coveney et al., in Rubber Chemistry and Technology: septembre 1995, Vol. 68, No. 4, pp. 660-670,

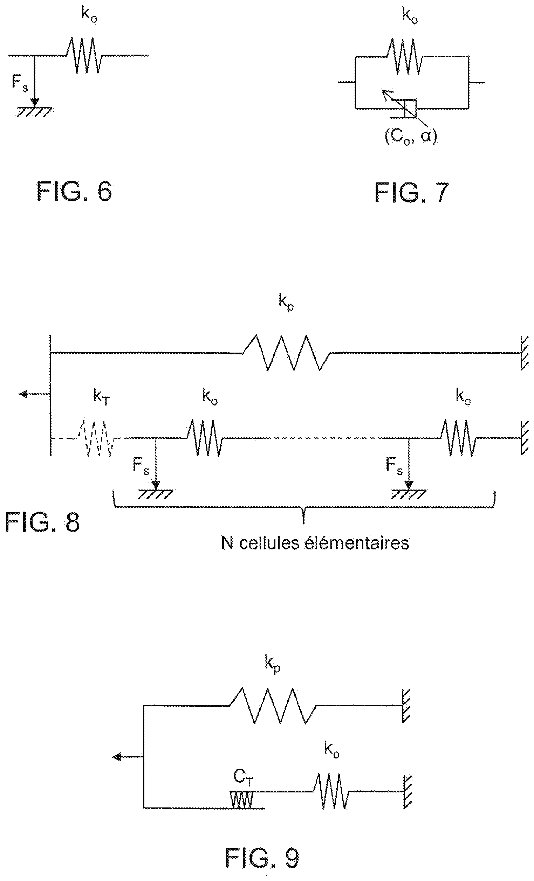

Ce modèle est fondé sur la combinaison en série d'un élément ayant un comportement purement élastique (représenté par un ressort) et d'un élément ayant un comportement tribologique (représenté par un élément de friction solide). Le résultat de la superposition linéaire de ces deux éléments est la cellule élémentaire. La figure 6 représente une cellule élémentaire avec un ressort de raideur $k_0$ et un élément de friction solide présentant une force seuil $F_s$. La valeur de la force seuil $F_s$ est une valeur limite, c'est-à-dire qu'une force appliquée à l'élément de friction solide de valeur supérieure à la valeur limite met l'élément de friction solide en mouvement translatoire et une force appliquée à l'élément de friction solide de valeur inférieure à la valeur limite n'entraine pas l'élément de friction solide en mouvement.

Il est possible de parfaire le modèle en utilisant une combinaison en série d'une pluralité de cellules élémentaires. Dans ce cas, un élément purement élastique supplémentaire est nécessaire et est disposé en parallèle avec la combinaison en série de cellules élémentaires. Cet élément purement élastique supplémentaire, représenté par un ressort de raideur $k_p$, traduit une raideur élastique globale. La figure 8 représente un tel agencement des éléments mentionnés (sans le ressort $k_T$ en pointillés).

Il est encore possible de parfaire le modèle en ajoutant un autre élément purement élastique disposé en série avec la combinaison en série de cellules élémentaires. Cet autre élément purement élastique, représenté par un ressort de raideur $k_T$, traduit une raideur additionnelle à faible amplitude de déplacement (voir figure 8).

Il est encore possible de parfaire le modèle en ajoutant des cellules de Maxwell pour modéliser le comportement viscoélastique.

D'autres modèles sont encore possibles, comme par exemple le modèle triboélastique dépendant de la vitesse (ou en anglais *rate-dependent triboelastic model,* ou encore *RT model*) présenté par l'article « Rate-dependent modeling of a highly filled vulcanizate » (en français « Modélisation dépendante de la vitesse d'un vulcanisé hautement chargé »), de V. A. Coveney et D. E. Johnson, in Rubber Chemistry and Technology: septembre 2000, Vol. 73, No. 4, pp. 565-577. Dans ce modèle, l'élément de friction solide est remplacé par un élément de friction dépendant de la vitesse pour former la cellule élémentaire (voir la figure 7).

De manière générale, le modèle analytique fournit une équation décrivant la force appliquée F en fonction de la déformation x, des maxima de la force appliquée ($F_m$) et de la déformation ($X_m$), ainsi que des paramètres analytiques tels que $k_P$, $C_T$ et $K_O$... Le modèle analytique ne fournit que la forme générale de l'équation. Il convient alors de déterminer les paramètres analytiques.

Grâce au modèle STS, l'équation suivante par exemple peut être déterminée et décrit le comportement de la pièce face à un effort en compression :

$$F = k_P \cdot x + F_m + \frac{\lambda \cdot C_T}{2K_0} \cdot \text{sgn}(x - X_m) \cdot \left( \sqrt{1 + 4k_0^2 \cdot \frac{|x - X_m|}{\lambda \cdot C_T}} - 1 \right),$$

Avec $\lambda$=1 avant le premier extremum et 2 ensuite.

À partir des mesures effectuées lors de l'étape c) (voir figure 4) et le modèle analytique, les paramètres analytiques peuvent être déterminés ($K_P$, $C_T$ et $K_O$).

Ainsi, l'équation fournie par le modèle analytique peut être complétée pour la pièce donnée et son comportement quasi-statique sous différentes conditions de traction/compression peut être déduit de l'équation complétée (voir figure 5).

La raideur quasi-statique est obtenue par la formule suivante :

$$k_s = \frac{F_2 - F_1}{X_2 - X_1},$$

où $k_s$ est la raideur quasi-statique, $F_1$ et $F_2$ deux valeurs de la force appliquée dans une zone quasi-linéaire des courbes obtenues grâce au modèle analytique complété et X1 et X2, les déformations correspondantes.

La phase quasi-statique est calculée à partir de l'aire à l'intérieur de la courbe correspondante.

Une comparaison de la valeur de la raideur quasi-statique, respectivement de celle de la phase quasi-statique, avec une valeur de raideur quasi-statique, respectivement de phase quasi-statique, prédéterminée est réalisée. Si la valeur de la raideur quasi-statique, respectivement de la phase quasi-statique, est égale à la valeur de raideur quasi-statique, respectivement de phase quasi-statique, prédéterminée plus ou moins une tolérance que le cahier des charges considère comme acceptable, alors la pièce remplit les conditions de raideur quasi-statique, respectivement de phase quasi-statique, et elle est déclarée conforme. Sinon, la pièce est déclarée non conforme et est mise au rebut.

Dans le cas où les deux valeurs sont utilisées pour la détermination de la conformité de la pièce, il faut que la pièce remplisse les critères prédéfinis pour les deux valeurs afin d'être déclarée conforme, sinon elle est déclarée non conforme.

La détermination de la conformité de la pièce comprend avantageusement la détermination de la valeur d'une raideur dynamique correspondant à celle qui aurait été déterminée en appliquant à la pièce une force de compression et une force de traction de manière répétée, notamment de manière périodique à une fréquence f. Dans ce cas, la valeur de la raideur dynamique est comparée à une valeur de raideur dynamique prédéterminée. Si la valeur de la raideur dynamique est égale à la valeur de raideur dynamique prédéterminée, plus ou moins une tolérance que le cahier des charges considère comme acceptable, alors la pièce remplit les conditions de raideur dynamique. La pièce est déclarée conforme si elle remplit toutes les conditions fixées, sinon elle est déclarée non conforme.

La raideur dynamique à la fréquence correspondant au profil de déformation utilisé pendant l'étape b) est obtenue par la formule suivante :

$$k_d = \frac{F_m}{X_m},$$

où $k_d$ est la raideur dynamique à cette fréquence, $F_m$ l'amplitude maximale de la force appliquée et $X_m$ la déformation correspondante.

De préférence, le profil de déformation utilisé à l'étape b) est quasi-linéaire et la fréquence f répond à la formule :

$$f = \frac{v}{A} ;$$

avec v la vitesse de déformation utilisée à l'étape b) et A l'amplitude maximale de la force de compression appliquée à l'étape b).

De manière générale, selon la pièce fabriquée, le cahier des charges qui a été établi réclame une caractérisation en dynamique de la pièce. Il est donc nécessaire de déterminer une valeur d'une caractéristique mécanique dynamique intrinsèque de la pièce relative à la réponse de la pièce à une sollicitation dynamique selon un profil dynamique prédéfini de déformation de la pièce. Cette détermination est réalisée à partir de la mesure de la déformation de la pièce et de la force de compression appliquée.

Lorsque les caractéristiques mécaniques quasi-statiques déterminées sont la raideur quasi-statique et/ou la phase quasi-statique, la caractéristique mécanique dynamique est de préférence la raideur dynamique et/ou la phase dynamique correspondant à des paramètres représentatifs de la réponse mécanique de la pièce à une excitation de la pièce à une fréquence donnée. L'une est l'autre sont déterminées à partir de la raideur quasi-statique et/ou la phase quasi-statique.

Par exemple, celles-ci peuvent être obtenues dans le présent procédé grâce à des coefficients de durcissement en fonction de la fréquence. Ces coefficients traduisent le phénomène observé de durcissement des élastomères chargés lorsque ceux-ci subissent des efforts de compression et de traction à des fréquences élevées.

Le terme de « fréquence » utilisé ici fait référence au fait que dans les procédés conventionnels, la pièce est soumise à une répétition de cycles comprenant l'application d'une force de compression et d'une force de traction. Cette répétition donne lieu à une déformation de la pièce sous forme sinusoïdale à une fréquence donnée. Ainsi, le présent procédé permet de déterminer la raideur dynamique et la phase dynamique que l'on aurait obtenues par les procédés conventionnels à une fréquence donnée. Ces fréquences sont généralement comprises entre 1 Hz et 300 Hz.

La raideur dynamique $k_f$ à une fréquence f est donnée par la formule suivante :

$$k_f = C_{k,f} \cdot k_d ;$$

$k_d$ étant la raideur dynamique obtenue ci-dessus et $C_{k,f}$ le coefficient de durcissement en fonction de la fréquence pour la raideur.

La phase dynamique $\varphi_k$ à la fréquence f est donnée par la formule suivante :

$$\varphi_f = C_{\varphi,k} \cdot \varphi ;$$

$\varphi$ étant la phase obtenue par le procédé ne formant pas partie de l'invention et $C_{\varphi,f}$ le coefficient de durcissement en fonction de la fréquence pour la phase.

Les coefficients de durcissement en fonction de la fréquence dépendent du caoutchouc utilisé et du profil de déformation utilisé pendant l'étape b). Ces coefficients peuvent être déterminés à partir de mesures dynamiques conventionnelles ou base de données élastomère interne. Ils peuvent également être obtenus durant la mesure par d'autres méthodes (par exemple par fluage ou relaxation) ; cependant ces dernières méthodes s'avèrent plus longues.

Le procédé de contrôle qualité décrit ci-dessus peut être intégré à un procédé de fabrication et de contrôle d'une pièce au moins partiellement en élastomère chargé, notamment en caoutchouc. Ce procédé comprend :

- la fabrication de la pièce au moins partiellement en élastomère chargé ; et
- le contrôle qualité de la pièce fabriquée à l'aide du procédé de contrôle qualité décrit ci-dessus.

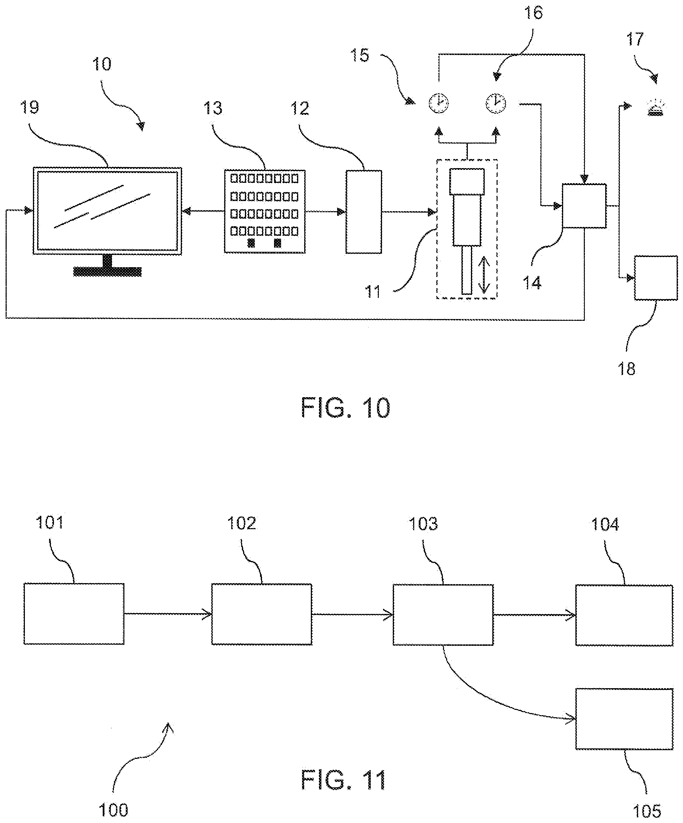

Un exemple de machine d'essai spécialement dédiée à la mise en œuvre du procédé décrit ci-dessus est décrit en référence à la figure 10.

La machine d'essai **10** comprend une cellule **11** d'application d'efforts pour appliquer la force de compression sur la pièce, une commande **12** pour commander la cellule **11** d'application d'effort, un régulateur **13**, de préférence programmable, pour régler la commande **12**, un calculateur **14**, un capteur de charge **15** pour la mesure de la force appliquée et un capteur de déplacement **16** pour la mesure de la déformation de la pièce.

Le capteur de charge **15** et le capteur de déplacement **16** sont connectés d'un côté à la cellule **11** d'application d'efforts et de l'autre au calculateur **14**.

Le régulateur **13** règle la commande **12** pour faire effectuer à la cellule **11** d'application d'efforts un cycle d'accommodation de la pièce consistant à l'application d'une force de compression progressive sur la pièce et le relâchement progressif sans traction de la force de compression.

Le régulateur **13** règle la commande **12** pour également faire effectuer à la cellule **11** d'application d'effort l'application d'une force de compression progressive sur la pièce en régime quasi-statique à une vitesse de déformation de la pièce donnée.

Le capteur de charge **15** et le capteur de déplacement **16** sont réglés pour effectuer les mesures pendant l'application de la force de compression par la cellule **11** d'application d'efforts et pour envoyer ces mesures au calculateur **14**.

Le calculateur **14** est apte à déterminer la conformité de la pièce à partir de la mesure de la déformation de la pièce et de la force de compression appliquée.

La cellule **11** d'application comprend de préférence un vérin électrique.

La machine d'essai **10** peut également comprendre un signaleur **17** connecté au calculateur **14** pour émettre un signal lorsqu'il a été déterminé que la pièce ne répondait pas aux exigences de qualité. Le signal peut être visuel, tel l'allumage d'une lampe, ou sonore, tel l'émission d'un son.

La machine d'essai **10** peut en outre ou alternativement comprendre un aiguillage **18** connecté au calculateur pour diriger la pièce détectée comme défectueuse vers un bac de rebus.

La machine d'essai **10** peut encore comprendre un afficheur **19,** tel un écran d'ordinateur, une tablette, un écran LCD, connecté au calculateur **14** pour afficher les caractéristiques de la pièce qui ont été mesurées. L'afficheur **19** peut également afficher d'autres informations telles que le numéro de lot, la conformité ou non au cahier des charges, etc. L'afficheur **19** peut également être connecté au régulateur **13** afin de faciliter le réglage de la commande.

Ainsi, la machine d'essai **10** est adaptée pour mettre en oeuvre le procédé de contrôle qualité décrit ci-dessus.

Cette machine d'essai **10** peut être intégrée à une ligne **100** de fabrication de pièces au moins partiellement en élastomère chargé (figure 11), comprenant en outre :

- une station **101** de fabrication des pièces ;

- une station **102** de contrôle qualité des pièces comportant la machine d'essai décrite **10** ci-dessus ; et

- une station **103** d'aiguillage pour séparer les pièces ne remplissant pas les critères du contrôle qualité des pièces remplissant les critères du contrôle qualité.

Éventuellement, la ligne **100** de fabrication peut également comprendre un bac de récupération **104** destiné à recevoir les pièces ayant passé le contrôle qualité et un bac de rebut **105** destiné à recevoir les pièces n'ayant pas passé avec succès le contrôle qualité.

Exemple 1

Le présent procédé a été testé sur une partie molle de 10 pièces d'une référence en caoutchouc. Ces pièces sont des articulations type support d'amortisseur, articulations souple. 1 cycle d'accommodation a été réalisé. Suite au cycle d'accommodation, les pièces ont été soumises à une force de compression progressive jusqu'à une amplitude maximale de 780 N. La vitesse de déformation a été fixée à 1 mm/s.

Le modèle STS décrit ci-dessus a été utilisé pour déterminer la raideur et la phase de la pièce.

Les résultats dans le tableau 1 suivant ont été obtenus. Ces résultats sont comparés aux résultats obtenus avec des méthodes conventionnelles en régime stationnaire et dynamique utilisant une machine servohydraulique.

Tableau 1

| | Exemple 1 | Méthodes conventionnelles | Différences (en pourcentage) |
|---|---|---|---|
| **Raideur (N/mm)** | | | |
| Quasi-statique, $\pm$ 1,00 mm* | 445 | 453 | 1,7 |
| Dynamique, $\pm$ 1,00 mm, 1 Hz* | 479 | 477 | 0,5 |
| Dynamique, $\pm$ 1,00 mm, 15 Hz | 497 | 500 | 0,6 |
| Dynamique, $\pm$ 0,10 mm, 150 Hz | 967 | 936 | 3,3 |
| | | | |
| **Phase (degrés)** | | | **Différences en degrés** |
| $\pm$ 1,00 mm, 1 Hz* | 7,95 | 7,8 | 0,15 |
| $\pm$ 1,00, 15 Hz | 9,05 | 9 | 0,05 |

Les données marquées d'un astérisque suffisent au contrôle qualité.

Dans le domaine de la caractérisation mécanique de caoutchouc, l'erreur de mesure en valeur absolue est généralement de l'ordre de 5 %. Ainsi, une détermination présentant une différence inférieure à 5 % à celle des méthodes conventionnelles est très satisfaisante.

Exemple 2

Le présent procédé a été testé sur une partie molle de 3 pièces d'une référence en caoutchouc. Ces pièces sont des articulations type articulations élastiques cylindriques, articulations rigides. 1 cycle d'accommodation a été réalisé. Suite au cycle d'accommodation, les pièces ont été soumises à une force de compression progressive jusqu'à une amplitude maximale de 6500N. La vitesse de déformation a été fixée à 0.5 mm/s.

Le modèle STS décrit ci-dessus a été utilisé pour déterminer la raideur et la phase de la pièce.

Les résultats dans le tableau 2 suivant ont été obtenus. Ces résultats sont comparés aux résultats obtenus avec des méthodes conventionnelles en régime stationnaire et dynamique utilisant une machine servo-hydraulique.

Tableau 2

| | Exemple 2 | Méthodes conventionnelles | Différences (en pourcentage) |
|---|---|---|---|
| Raideur | | | |
| Quasi-statique, ± 0,30 mm* | 15813 | 15968 | 0,97 |
| Dynamique, ± 0,10 mm, 1 Hz* | 21137 | 21233 | 0,45 |
| Dynamique, ± 0,01 mm, 100 Hz | 35661 | 35453 | 0,59 |
| | | | |
| Phase | | | Différences en degrés |
| ± 0,10 mm, 1 Hz* | 7,58 | 6,86 | 0,71 |

Les données marquées d'un astérisque suffisent au contrôle qualité.

Dans le domaine de la caractérisation mécanique de caoutchouc, l'erreur de mesure en valeur absolue est généralement de l'ordre de 5 %. Ainsi, une détermination présentant une différence inférieure à 5 % à celle des méthodes conventionnelles est très satisfaisante.

## Revendications

1. Machine (10) d'essai, spécialement dédiée à la mise en œuvre d'un procédé de contrôle qualité d'une pièce au moins en élastomère chargé, moyennant l'application d'une force de compression progressive sur la pièce en régime quasi-statique jusqu'à atteindre une amplitude maximale de déformation de la pièce, cette machine (10) comprenant :

   - une cellule (11) d'application d'efforts, pour appliquer la force de compression sur la pièce, qui comprend un vérin (3) électrique,
   - une commande (12) pour commander la cellule (11) d'application d'efforts,
   - un régulateur (13) programmable, pour régler la commande (12),
   - un capteur (15) de charge connecté d'un côté à la cellule (11) d'application d'efforts pour la mesure de la force appliquée pendant l'application de la force de compression par la cellule (11) d'application d'efforts,
   - un capteur (16) de déplacement connecté d'un côté à la cellule (11) d'application d'efforts pour la mesure de la déformation de la pièce pendant l'application de la force de compression par la cellule (11) d'application d'efforts,
   - le capteur (15) de charge et le capteur (16) de déplacement étant connectés de l'autre côté à un calculateur (14), apte à déterminer la conformité de la pièce à partir de la mesure de la déformation et de la force de compression appliquées,

   **caractérisée :**

   - **par** un logement de la machine (10) dans lequel la pièce est disposée sans fixation,
   - en ce que le régulateur (13) règle la commande (12) pour faire effectuer à la cellule (11) d'application d'efforts un cycle d'accommodation de la pièce consistant à l'application de la force de compression progressive sur la pièce et le relâchement progressif sans traction de la force de compression,
   - en ce que l'amplitude maximale de déformation de la pièce se situe dans un domaine où la relation entre la force appliquée et la déformation est linéaire, et
   - en ce que le calculateur (14) est apte à déterminer la conformité de la pièce à partir de la mesure de la déformation de la pièce et de la force de compression appliquée, la conformité étant déterminée selon le modèle du solide triboélastique standard (STS).

**Patentansprüche**

1. Prüfmaschine (10), die insbesondere für die Durchführung eines Qualitätskontrollverfahrens an mindestens einem Werkstück aus gefülltem Elastomer bestimmt ist, indem auf das Werkstück eine progressive Druckkraft in quasistatischer Regelung ausgeübt wird, bis eine maximale Verformungsamplitude des Werkstücks erreicht ist, wobei diese Maschine (10) folgendes umfasst:

   - eine Einheit (11) zur Anwendung der Druckkraft auf das Werkstück, die einen Elektrozylinder (3) umfasst,
   - eine Steuerung (12) zur Bedienung der Kraftanwendungseinheit (11),
   - einen programmierbaren Regler (13) zur Einstellung der Steuerung (12),
   - einen Lastsensor (15), der an einer Seite mit der Kraftanwendungseinheit (11) verbunden ist, um die angewandte Kraft während der Anwendung der Druckkraft durch die Kraftanwendungszelle (11) zu messen,
   - einen Wegsensor (16), der an einer Seite mit der Kraftanwendungseinheit (11) verbunden ist, um die Verformung des Werkstücks während der Anwendung der Druckkraft durch die Kraftanwendungseinheit (11) zu messen,
   - wobei der Lastsensor (15) und der Wegsensor (16) auf der anderen Seite mit einem Rechner (14) verbunden sind, der in der Lage ist, die Konformität des Werkstücks anhand der Messung der Verformung und der angewandten Druckkraft zu bestimmen,

   **dadurch gekennzeichnet:**

   - **dass** das Werkstück ohne Befestigung in einer Aufnahme der Maschine (10) angeordnet ist,
   - **dass** der Regler (13) die Steuerung (12) so einstellt, dass die Kraftanwendungseinheit (11) einen Werkstückverhaltenszyklus durchführt, der aus der Anwendung der progressiven Druckkraft auf das Werkstück und der zugfreien progressiven Lockerung der Druckkraft besteht,
   - **dass** die maximale Verformungsamplitude des Werkstücks in einem Bereich liegt, in dem die Beziehung zwischen der angewandten Kraft und der Verformung linear ist, und
   - dadurch, dass der Rechner (14) in der Lage ist, die Konformität des Werkstücks anhand der Messung der Verformung des Werkstücks und der angewandten Druckkraft zu bestimmen, wobei die Konformität nach dem Modell des triboelastischen Festkörper-Standards (STS) bestimmt wird.

**Claims**

1. A testing machine (10), specifically dedicated to the implementation of a method for controlling the quality of a part at least partially made of filled elastomer, through the application of a gradual compressive force onto the part with a quasi-static regime until a maximum strain of the part is reached, with such machine (10) comprising:

   - a stress application cell (11), for applying the compressive force onto the part, which comprises an electric jack (3),
   - a control (12) for controlling the stress application cell (11),
   - a programmable regulator (13), for regulating the control (12),
   - a filler sensor (15) connected, on one side, to the stress application cell (11) for measuring the force applied when the stress application cell (11) applies the compressive force,
   - a position sensor (16) connected, on one side, to the stress application cell (11) for measuring the force applied when the stress application cell (11) applies the compressive force,
   - with the filler sensor (15) and the position sensor (16) being connected on the other side to a computer (14), able to determine the conformity of the part from the measurement of the applied strain and the compressive force, **characterized:**
   - **by** a recess in the machine (10) wherein the part is positioned but not fixed,
   - in that the regulator (13) regulates the control (12) to make the stress application cell (11) execute a part accommodation cycle consisting in applying the gradual compressive force onto the part and the gradual release of the compressive force with no traction,
   - in that the maximum strain of the part occurs in a field where the relationship between the applied force and the strain is linear, and
   - in that the computer (14) is able to determine the conformity of the part from the measurement of the part strain and the applied compressive force, with the conformity being determined according to the Standard Triboelastic Solid (STS) model .

```
┌─────────────────────────────┐
│    fabrication d'une pièce   │
└─────────────────────────────┘
               │
               ▼
┌─────────────────────────────┐
│   cycle(s) d'accommodation   │
└─────────────────────────────┘
               │
               ▼
┌─────────────────────────────┐
│     application d'une force  │
│        de compression        │
└─────────────────────────────┘
               │
               ▼
┌─────────────────────────────┐
│   mesure de la déformation   │
│  et de la force de compression│
└─────────────────────────────┘
               │
               ▼                        ┌──────────────────────────────┐
┌─────────────────────────────┐         │       modèle analytique      │
│      détermination de la     │         └──────────────────────────────┘
│      conformité de la pièce  │
└─────────────────────────────┘         ┌──────────────────────────────┐
                                         │ Comparaison avec les besoins │
                                         │    du cahier des charges     │
                                         └──────────────────────────────┘
```

FIG. 1

FIG. 2

FIG. 3

FIG. 4

FIG. 5

$k_o$

$F_s$

FIG. 6

$k_o$

$(C_o, \alpha)$

FIG. 7

$k_p$

$k_T$        $k_o$                    $k_o$

$F_s$                    $F_s$

FIG. 8

N cellules élémentaires

$k_p$

$k_o$

$C_T$

FIG. 9

FIG. 10

FIG. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012080675 A

- FR 2925691

**Littérature non-brevet citée dans la description**

- A triboelastic Model for the Syclic Mechanical Behavior of Filled Vulcanizates » et « Rate-dependent modeling of a highly filled vulcanizate. **COVENEY V A et al.** RUBBER CHEMISTRY AND TECHNOLOGY. AMERICAN SOCIETY, 01 Septembre 1995, vol. 68, 660-670
- RUBBER CHEMISTRY AND TECHNOLOGY. 01 Septembre 2000, vol. 73, 565-577

- **V. A. COVENEY et al.** A triboelastic model for the cyclic mechanical behavior of filled vulcanizates » (en français « Un modèle triboélastique pour le comportement mécanique cyclique des vulcanisés chargés). *Rubber Chemistry and Technology,* Septembre 1995, vol. 68 (4), 660-670
- **V. A. COVENEY ; D. E. JOHNSON.** « Rate-dependent modeling of a highly filled vulcanizate » (en français « Modélisation dépendante de la vitesse d'un vulcanisé hautement chargé »). *Rubber Chemistry and Technology,* Septembre 2000, vol. 73 (4), 565-577